(19) Europäisches Patentamt / European Patent Office / Office européen des brevets

(11) **EP 3 785 554 B1**

(12) **EUROPEAN PATENT SPECIFICATION**

(45) Date of publication and mention of the grant of the patent:
**25.06.2025 Bulletin 2025/26**

(21) Application number: **19791863.4**

(22) Date of filing: **19.04.2019**

(51) International Patent Classification (IPC):
**A24F 40/40** *(2020.01)* **H05B 1/02** *(2006.01)*

(52) Cooperative Patent Classification (CPC):
**A24F 40/53; A24F 40/40; H05B 1/02; A24F 40/20**

(86) International application number:
**PCT/KR2019/004761**

(87) International publication number:
**WO 2019/208974 (31.10.2019 Gazette 2019/44)**

(54) **AEROSOL GENERATION DEVICE**

AEROSOLERZEUGUNGSVORRICHTUNG

DISPOSITIF DE GÉNÉRATION D'AÉROSOL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2018 KR 20180047816**

(43) Date of publication of application:
**03.03.2021 Bulletin 2021/09**

(73) Proprietor: **KT&G Corporation**
**Daedeok-gu**
**Daejeon 34337 (KR)**

(72) Inventors:
• **JUNG, Sun Hwan**
**Daejeon 34337 (KR)**
• **KO, Dong Kyun**
**Sejong 30098 (KR)**
• **PARK, In Su**
**Seoul 07529 (KR)**
• **YANG, Ji Hun**
**Sejong 30126 (KR)**
• **JEOUNG, Eun Mi**
**Daejeon 34308 (KR)**

(74) Representative: **Ter Meer Steinmeister & Partner**
**Patentanwälte mbB**
**Nymphenburger Straße 4**
**80335 München (DE)**

(56) References cited:
WO-A1-2016/199066        WO-A1-2018/050701
KR-A- 20160 111 902      KR-A- 20170 106 915
KR-A- 20180 033 141      KR-B1- 100 579 783
US-A1- 2015 013 696      US-A1- 2017 172 215
US-A1- 2018 049 469      US-A1- 2018 049 472

## Description

[Technical Field]

The present invention relates to an aerosol generating apparatus.

[Background Art]

In recent years, there is an increasing demand for alternative methods to overcome the shortcomings of general cigarettes. For example, there is an increasing demand for a method for generating an aerosol by heating an aerosol generating material in a cigarette instead of a method for generating an aerosol by combusting the cigarette. Accordingly, researches on a heating-type cigarette or a heating-type aerosol generating apparatus have been actively conducted.

An aerosol generating apparatus is a new type of smoking apparatus, and forms a new market of replacing conventional cigarettes. The aerosol generating apparatus includes a liquid nicotine vaporizing device for vaporizing a liquid tobacco or an aerosol generating apparatus for generating smoking gas by a fumigation method by heating a cigarette.

When using an aerosol generating apparatus having a heater that heats a cigarette by using electricity, the cigarette which is heated by the heater to generate the smoking gas is separated and discarded from the aerosol generating apparatus and then a new cigarette may be inserted into the aerosol generating apparatus.

Korean Patent Registration No. 10-1667124 relates to an aerosol generating apparatus for generating smoking gas by heating a cigarette, and describes a structure of a holder that assists an operation of inserting the cigarette into the aerosol generating apparatus or separating the cigarette from the aerosol generating apparatus.

When a user uses the aerosol generating apparatus having such a structure, an operation of pushing the holder and the cigarette to the aerosol generating apparatus by inserting the cigarette into the holder that is extracted to the outside of the aerosol generating apparatus is performed during smoking, and then a preliminary operation of pressing a heating button is required to start heating. In addition, in order to terminate smoking before a predetermined heating time, when the cigarette is removed, the heating button needs to be pressed to stop heating, and if the button is not pressed, heating is continued for a predetermined heating time by an internal timer.

As described above, since a conventional aerosol generating apparatus does not determine whether or not a cigarette is inserted, an operation of pressing the heating button is required for starting and ending heating. In addition, even when the cigarette is not inserted, the heating button may be pressed and the heater is heated due to user's carelessness, and if a heating end button is not pressed, the heater is continuously heated, and thus, there is a problem of unnecessary battery consumption.

[Prior Art Document]

[Patent Document]

Korean Patent Registration No. 10-1667124
US 2017/172215 A1 presents a cigarette heating device for heating a cigarette includes a main sleeve unit, a central heater, a movable element, an internal cup, and a restoring element. The main sleeve unit presets a first position and a second position along an axial direction. The movable element is movable between the first position and the second position. The internal cup is configured for receiving the cigarette. The internal cup is in linkage relation with the movable element. When the movable element is in the first position, the internal cup is adjacent to the central heater, so that the central heater heats the cigarette. When the movable element is in the second position, the internal cup is away from the central heater, so that the central heater is separated from the cigarette. The restoring element is configured for driving the movable element to restore from the second position to the first position.

WO 2016/199066 A1 presents a system that includes a container housing an aerosol generating substrate and a product identifying compound associated with the container. The system further includes an electronic article configured to receive the container. The electronic article includes a sensor configured to detect the product identifying compound.

[Disclosure]

[Technical Problem]

As described above, in order to solve the problems of the conventional aerosol generating apparatus, it is an object of the present invention to provide an aerosol generating apparatus in which a heater is heated when a cigarette is inserted by detecting whether the cigarette is inserted or not and the heating of the heater is terminated when the cigarette is removed

[Technical Solution]

In order to achieve the above object, the present invention provides an aerosol generating apparatus as set out in independent claim 1.

[Advantageous Effects]

The aerosol generating apparatus of the present invention may automatically heat or terminate a heater by detecting whether or not a cigarette is inserted, and thus, a preliminary operation of pressing a heating button is not required, thereby increasing the user's convenience and may prevent an operation of pressing the heating button due to the user's carelessness, thereby reducing unnecessary battery consumption.

[Description of Drawings]

FIG. 1 is a perspective view exemplarily illustrating an operating state of an aerosol generating apparatus according to an embodiment.

FIG. 2 is a cross-sectional view illustrating a state in which a cigarette is not inserted into the aerosol generating apparatus.

FIG. 3 is a cross-sectional view illustrating a state in which the cigarette is not inserted into the aerosol generating apparatus.

FIG. 4 is a cross-sectional view illustrating an operating state in which the cigarette is inserted into the aerosol generating apparatus.

FIG. 5 is a cross-sectional view enlarging and illustrating a part of the aerosol generating apparatus in an operating state for inserting the cigarette or in an operating state of removing the cigarette.

FIG. 6 is a cross-sectional view enlarging and illustrating a part of the aerosol generating apparatus in an operating state for inserting the cigarette or in an operating state of removing the cigarette.

FIG. 7 is a cross-sectional view illustrating a state in which a cigarette is not inserted into an aerosol generating apparatus according to another embodiment.

FIG. 8 is a cross-sectional view illustrating a state in which the cigarette is not inserted into the aerosol generating apparatus according to another embodiment.

FIG. 9 is a cross-sectional view enlarging and illustrating a part of the aerosol generating apparatus according to the embodiment illustrated in FIGS. 7 and 8.

FIG. 10 is a cross-sectional view illustrating an operating state of the aerosol generating apparatus according to the embodiment illustrated in FIG. 9.

FIG. 11 is a configuration diagram illustrating an example of the aerosol generating apparatus.

FIGS. 12 and 13 are diagrams illustrating an example of a holder in various aspects.

FIG. 14 is a configuration diagram illustrating an example of a cradle.

[Best Mode]

Hereinafter, the present invention will be described in more detail.

The terms used in the embodiments were selected from general terms that are currently widely used as possible by considering functions in the present invention, but the terms may vary depending on an intention of those skilled in the art, a precedent, emergence of new technology, etc. Further, in a specific case, there are also terms arbitrarily selected by the applicant and in this case, the meaning of the terms will be described in detail in a corresponding description part of the invention. Accordingly, the term used in the present invention should be defined based on not just a name of the term but a meaning of the term and contents throughout the present invention.

Throughout the specification, when any part "comprises" a certain component, it is meant that other components may be further included instead of excluding other components, unless specifically stated otherwise. In addition, terms of "part', "module" and the like described in the specification mean a unit that processes at least one function or operation, which may be implemented in hardware or software or implemented in a combination of hardware and software.

Hereinafter, embodiments will be described in detail with reference to the accompanying drawings. However, the present invention may be embodied in many different forms and are not limited to embodiments described herein.

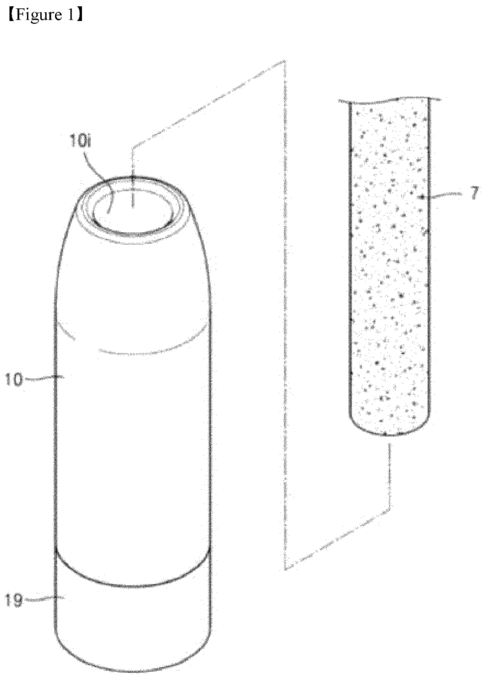

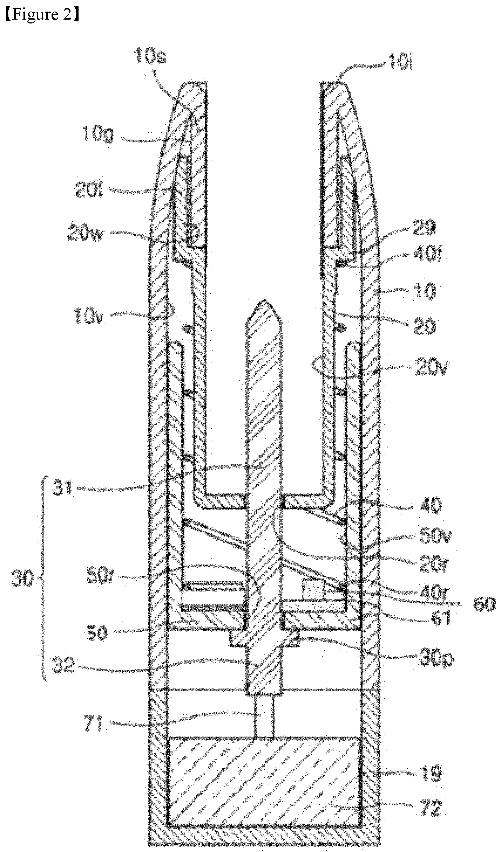

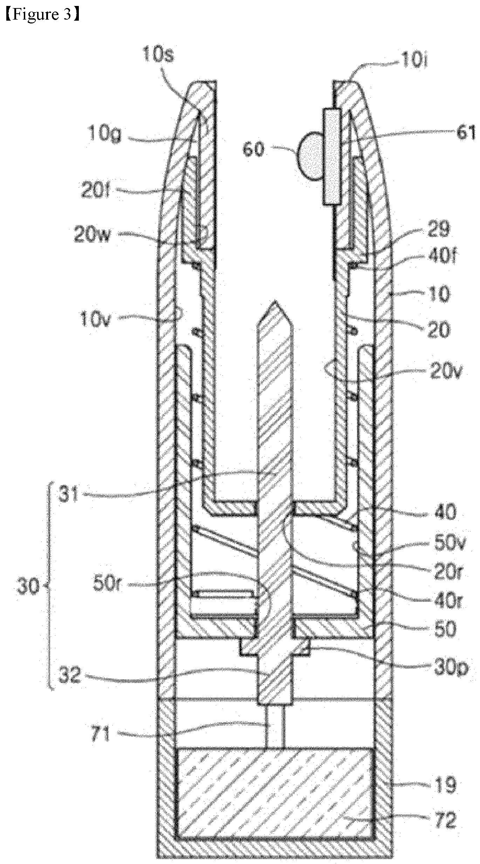

FIG. 1 is a perspective view exemplarily illustrating an operating state of an aerosol generating apparatus according to an embodiment and FIGS. 2 and 3 are cross-sectional views illustrating a state in which a cigarette is not inserted into the aerosol generating apparatus according to the embodiment illustrated in FIG. 1.

An aerosol generating apparatus according to the embodiment illustrated in FIGS. 1 to 3 includes a case 10, a container 20 installed inside the case 10 so as to be movable in a longitudinal direction of the case 10 and including a receiving space 20v receiving a cigarette 7, a heater 30 disposed inside the case 10 so that a front end 31 is inserted into the receiving space 20v of the container 20 and heating the cigarette 7, an elastic support part 40 supporting elastically the container 20 with respect to the case 10, and a switch 60 detecting whether the cigarette is inserted or not to apply electricity to the heater.

The case 10 forms an appearance of the aerosol generating apparatus and performs a function of receiving and protecting various components in an inner space 10v formed therein. The case 10 has a hollow cylindrical shape with an empty inside, and has an opening 10i into which the cigarette 7 may be inserted by opening to the outside at the front end 31. The case 10 may be made of a plastic material that does not transfer electricity and heat or a metallic material coated with a plastic material on the surface.

The container 20 is installed inside the case 10 so as to be movable in the longitudinal direction of the case 10. The cigarette 7 may be formed in a cylindrical shape, and the container 20 and the case 10 also extend in the longitudinal direction of the cigarette 7 in response to the shape of the cigarette 7.

The container 20 is formed in a hollow cylindrical shape, and includes an opening 10i opened in a front end so that the cigarette 7 is inserted, a through hole 20r through which the front end 31 of the heater 30 passes at a rear end, and a receiving space 20v receiving the cigarette 7 therein.

The container 20 receives and supports the cigarette 7 while received inside the case 10 and performs a function of moving in the longitudinal direction of the case 10 together with the cigarette 7. Accordingly, the container 20 is not separated from the outside of the case 10.

The heater 30 that performs a function of heating the cigarette 7 is disposed inside the case 10. The front end 31 of the heater 30 is inserted into the inside of the container 20 through the through hole 20r of the container 20, and when the cigarette 7 is received in the container 20, the front end 31 of the heater 30 is inserted into the rear end of the cigarette 7.

The size of the through hole 20r of the container 20 may correspond to the thickness of the front end 31 of the heater 30. For example, when the front end 31 of the heater 30 has a circular cross-section, the through hole 20r also has a circular cross-sectional shape, and an inner diameter of the through hole 20r is formed to correspond to an outer diameter of the front end 31 of the heater 30. Therefore, the inner surface of the through hole 20r maintains a state to be in contact with the outer surface of the front end 31 of the heater 30.

Accordingly, while the container 20 is moving, the through hole 20r may perform a function of scraping off a material attached to the outer surface of the front end 31 of the heater 30. Since the through hole 20r of the container 20 and the front end 31 of the heater 30 are in contact with each other and a relatively moving operation is repeatedly performed, a coating layer made of a wear-resistant material may be formed on the surface of the front end 31 of the heater 30. For example, the coating layer may include materials such as metal, alloy, ceramic, plastic, and glass.

The embodiment is not limited by the configuration in which the inner surface of the through hole 20r is in contact with the outer surface of the front end 31 of the heater 30 as described above. For example, the size of the through hole 20r is formed larger than the size of the front end 31 of the heater 30, so that the inner surface of the through hole 20r may be spaced apart from the outer surface of the front end 31 of the heater 30.

A rear end 32 of the heater 30 is electrically connected to an electricity supply device 72 disposed at the rear side of the case 10 through an electric wiring 71. A base 19 surrounding the electricity supply device 72 is connected to the rear end of the case 10. When the electricity of the electricity supply device 72 is supplied to the heater 30 while the cigarette 7 is inserted into the front end 31 of the heater 30, the heater 30 is heated to heat the cigarette 7.

FIGS. 2 and 3 illustrate a state in which the cigarette 7 is not inserted into the aerosol generating apparatus. In order for the cigarette 7 to be inserted into the aerosol generating apparatus and for the aerosol generating apparatus to perform a function of generating cigarette smoke, the cigarette 7 needs to be inserted to the position of the through hole 20r at the rear end of the container 20. A total length of the heater 30 is about 25 mm, and a length of a portion of the front end 31 of the heater 30 inserted into the cigarette 7 is about 12 mm. In this state, since the front end 31 of the heater 30 is inserted into the rear end of the cigarette 7, when the electricity is supplied to the heater 30, the heater 30 heats the cigarette 7 to generate the cigarette smoke.

The case 10 includes a fixing part 50 that is coupled to the rear end 32 of the heater 30 to fix the position of the heater 30 to the case 10. The fixing part 50 includes a space 50v having a hollow cylindrical shape with an opened upper end and receiving the container 20 therein. The fixing part 50 includes a hole 50r into which the front end 31 of the heater 30 is inserted at the rear end.

The front end 31 of the heater 30 passes through the hole 50r of the fixing part 50. The heater 30 has a flange 30p protruding from the outer surface. The position of the heater 30 may be fixed to the case 10 by fixing the flange 30p to the fixing part 50.

The elastic support part 40 is disposed between the fixing part 50 and the container 20 to perform a function of elastically supporting the container 20 with respect to the case 10. In the illustrated embodiment, the elastic support part 40 has been implemented by a cylindrical compression coil spring, but the embodiment is not limited to the example of the elastic support part 40. For example, the elastic support part 40 may be implemented by a compression cylinder using liquid or gas, rubber, or the like.

The container 20 includes an extension part 20f in which an inner diameter of one end is extended outward. The inner diameter of the extension part 20f is formed larger than the outer diameter of the cigarette 7. The case 10 includes an insertion part 10s which is inserted between an inner wall surface 20w of the extension part 20f of the container 20 and the outer circumferential surface of the cigarette 7 to extend linearly in a moving direction of the container 20. That is, while the extension part 20f of the container 20 is inserted into a guide space 10g formed between the extension part 20f of the case 10 and the inner wall surface 20w of the case 10, the container 20 may move linearly in a longitudinal direction of the case 10.

The container 20 includes a support projection 29 formed on a surface facing the fixing part 50 from the outside of the extension part 20f. The elastic support part 4 is disposed between the support projection 29 and the fixing part 50, and one end 40f of the elastic support part 40 is supported by the support projection 29 of the container 20 and the other end 40r thereof is supported by the fixing part 50.

The switch 60 includes a sensor 61 that receives an operation signal of the switch to apply electricity to the heater 30. The sensor 61 may be a micro controller unit (MCU), but is not limited thereto.

The switch 60 may be installed inside the case that guides the insertion of the cigarette when the cigarette is inserted, the inner wall surface of the container 20w, or a position pressed by a bottom surface of the container 20 at the time of completion of the movement of the container 20. More specifically, as illustrated in FIG. 2, the switch 60 may be installed between the fixing part 50 and the container 20 as illustrated in FIG. 2, or may be installed in the insertion part 10s to face the outer circumferential surface of the cigarette 7 as illustrated in FIG. 3.

In the present invention, the type of the switch 60 is not particularly limited, but for example, a push button switch, a tack switch, or the like may be used. As an example, when the switch 60 is installed between the fixing part 50 and the container 20 as illustrated in FIG. 2, the push button switch may be used, and when the switch 60 is installed in the insertion part 10s to face the outer circumferential surface of the cigarette 7 as illustrated in FIG. 3, the tack switch may be used.

The push button switch is a switch in which an on/off state is changed whenever the switch is pressed, and when the switch is in an on state by once pressing, the switch is maintained in the on state until the switch is pressed once again.

The tack switch is a switch in which an on state is maintained only when pressure is continuously applied to the switch, and is in an off state when no pressure is applied.

In order for the user to use the aerosol generating apparatus, the cigarette 7 needs to be inserted into the aerosol generating apparatus.

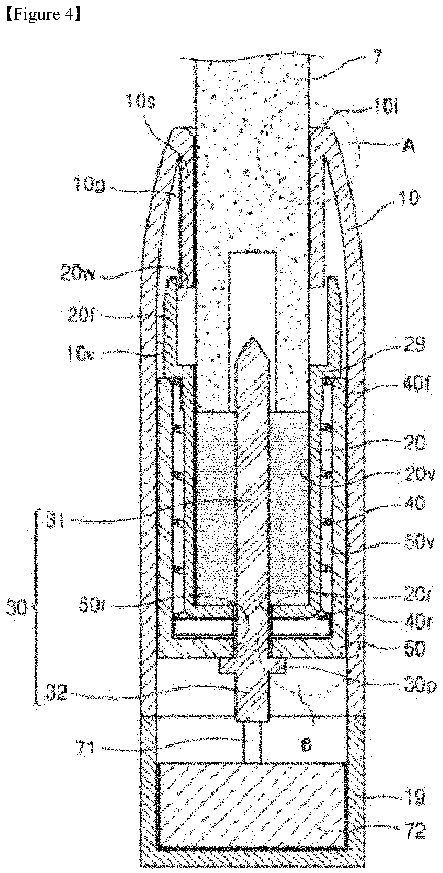

FIG. 4 illustrates an operating state in which the user presses the cigarette 7 to insert the cigarette 7 into the aerosol generating apparatus of FIGS. 2 and 3. When the user inserts the cigarette 7 into the receiving space 20v of the empty container 20 to insert the cigarette 7 into the aerosol generating apparatus and then presses the cigarette 7, the container 20 presses the elastic support part 40 together with the cigarette 7 and moves linearly toward the rear side as illustrated in FIG. 4 to move to the pressed position. While the cigarette 7 and the container 20 are pressed, the heater 30 is maintained to be fixed to the fixing part 50, and thus, the position of the heater 30 to the case 10 is not changed but maintained.

FIG. 4 is an operation for inserting the cigarette 7 into the aerosol generating apparatus, but even when the cigarette 7 is removed from the aerosol generating apparatus, the state may become an operation state similar to that illustrated in FIG. 4. When the cigarette 7 is pressed to remove the cigarette 7 from the aerosol generating apparatus, the container 20 presses the elastic support part 40 together with the cigarette 7 and moves toward the rear side.

The switch 60 is in an off state while the cigarette 7 is not inserted or the cigarette 7 is removed, and is in an on state while the cigarette 7 is inserted. When the switch 60 is in the on state, the sensor 61 receives an operation signal in the on state of the switch 60 and applies electricity to the heater 30 to heat the cigarette 7, and when the switch 60 is in the off state, the sensor 61 receives an operation signal in the off state of the switch 60 and stops the application of electricity to the heater 30 to terminate heating.

As described above, the switch 60 may be installed between the fixing part 50 and the container 20, or may be installed in the insertion part 10s to face the outer circumferential surface of the cigarette 7.

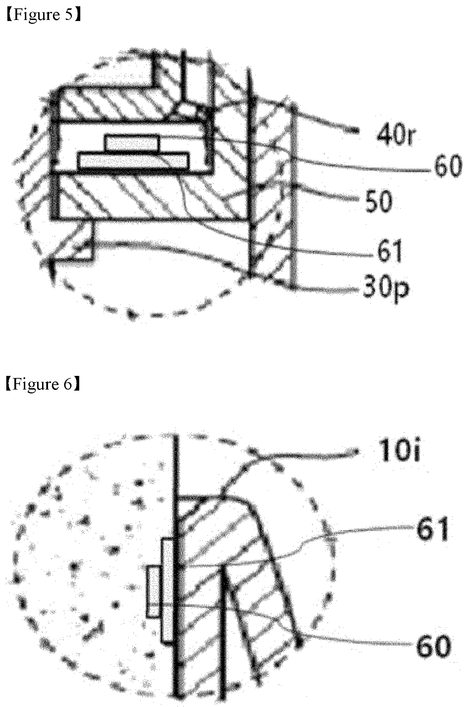

As an example, when the switch 60 is disposed between the fixing part 50 and the container 20 as illustrated in FIG. 2, the switch shows the off state while the cigarette 7 is not inserted and the sensor receives the off signal of the switch and does not apply the electricity to the heater. When the cigarette 7 is pressed to insert the cigarette 7, the container 20 presses the elastic support part 40 together with the cigarette 7, and the pressed elastic support part 40 presses the switch 60. Since the pressure is applied to the switch 60, the switch 60 detects the insertion of the cigarette 7 to be in the on state. The switch 60 in the on state means that the switch 60 is maintained in a pressed state, and is maintained in the on state until the switch 60 is pressed again. The sensor 61 receives the operation signal in the on state of the switch 60 and applies electricity to the heater 30 to heat the cigarette 7. Further, when the cigarette 7 is pressed again to remove the cigarette 7, the container 20 presses the elastic support part 40 together with the cigarette 7, and the pressed elastic support part 40 presses the switch 60 in the on state. Since the switch 60 in the on state is pressed again, the switch 60 detects the removal of the cigarette 7 to be in the off state, and the sensor 61 receives the operation signal in the off state of the switch 60 and stops the application of electricity to the heater 30 to stop the heating. In order to remove the cigarette 7, the switch 60 in the on state is pressed again to be in the off state, and the off state of the switch 60 means a state in which the switch 60 is not pressed. FIG. 5 illustrates a state for inserting or removing the cigarette 7, which is a state in which the switch 60 is pressed.

As an example, when the switch 60 is disposed in the insertion part 10s to face the outer circumferential surface of the cigarette 7 as illustrated in FIG. 3, the switch shows the off state while the cigarette 7 is not inserted and the sensor receives the off signal of the switch and does not apply the electricity to the heater. When the cigarette 7 is pressed to insert the cigarette 7, the outer circumferential surface of the cigarette 7 presses the switch 60 located in the insertion part 10s to face the outer circumferential surface of the cigarette 7. Since the pressure is applied to the switch 60, the switch 60 detects the insertion of the cigarette 7 to be in the on state. The switch 60 in the on state means a state in which pressure is continuously applied to the switch 60 by the cigarette 7. The sensor 61 receives the operation signal in the on state of the switch 60 and applies electricity to the heater 30 to heat the cigarette 7. In addition, when the cigarette 7 is removed by pressing the cigarette 7, the pressure applied to the switch 60 is released, and thus, the switch 60 detects the removal of the cigarette 7 to be in the off state, and the sensor 61 receives the operation signal in the off state of the switch 60 and stops the application of electricity to the heater 30 to stop the heating. The off state of the switch 60 means a state in which the switch 60 is not pressed. FIG. 6 illustrates a state for inserting or removing the cigarette 7, which is a state in which the switch 60 is pressed.

The switch 60 included in the aerosol generating apparatus of the present invention detects whether or not the cigarette 7 is inserted, and the sensor receives the detected operation signal to apply electricity to the heater for heating or to stop the application of electricity to terminate heating. In the conventional aerosol generating apparatus, a preliminary operation of pressing a separate heating button when starting or ending heating is necessarily required, but in the present invention, whether the cigarette is inserted or not is detected to automatically perform heating and heating termination of the heater and the preliminary operation is not required, thereby increasing convenience in use. In addition, it is possible to prevent an operation of pressing the heating button due to the user's carelessness, thereby reducing unnecessary battery consumption.

While the cigarette 7 is heated by the heater 30, a tobacco material (residue) generated in the cigarette 7 is condensed and attached to a contact surface between the heater 30 and the cigarette 7. As the cigarette 7 and the container 20 are pressed, the heater 30 maintains its position while the cigarette 7 and the container 20 move to the pressing position by linear motion as illustrated in FIG. 4. The container 20 moves by about 5 mm by moving to the pressing position. Due to this operation, the contact surface between the cigarette 7 and the heater 30, which has been maintained to be attached to each other by the tobacco material attached to the contact surface between the cigarette 7 and the heater 30, may be easily separated.

In the state illustrated in FIG. 4, if the user releases the cigarette 7 and the container 20 without pressing, the container 20 and the cigarette 7 move linearly toward to the front side by the restoring force of the elastic support part 40 as illustrated in FIGS. 2 and 3 and then the container 20 moves to an initial position, which is an original position, as illustrated in FIGS. 2 and 3. Thereafter, the user may completely separate the used cigarette 7 from the container 20 of the aerosol generating apparatus by grabbing the cigarette 7 and extracting the cigarette 7 out of the receiving space 20v of the container 20.

In a conventional aerosol generating apparatus, when the cigarette is separated from the aerosol generating apparatus, the user simply removes the cigarette from the aerosol generating apparatus, and thus, the tobacco material existing between the cigarette and the heater is often attached to the heater.

However, in the aerosol generating apparatus according to the above-described embodiment, before separating the cigarette 7 from the aerosol generating apparatus, the cigarette 7 and the container 20 may first move to the pressing position, that is, to a position moving downward so that container 20 presses the elastic support part 40 to the end as illustrated in FIG. 4.

While the container 20 and the cigarette 7 move to the pressing position, the position of the heater 30 to the case 10 is not changed by maintaining the heater 30 in the fixed state, so that the contact surface between the heater 30 and the cigarette 7 which have been fixed by the tobacco material may be easily separated.

In addition, by the operation in which the container 20 and the cigarette 7 move to the pressing position illustrated in FIG. 4 and the operation in which the container 20 and the cigarette 7 that have moved to the pressing position move to the initial positions illustrated in FIGS. 2 and 3, the operation of separating the tobacco material which has been attached to the surface of the heater 30 from the heater 30 occurs. That is, while the container 20 and the cigarette 7 reach the pressing position illustrated in FIG. 4, the cigarette 7 pushes the surface of the heater 30 to push out the residue attached to the surface of the heater 30. While the container 20 pressed by the elastic support part 40 moves to the initial position illustrated in FIGS. 2 and 3 again, the container 20 and the cigarette 7 vibrates by the pressing force of the elastic support part 40, and thus the vibration is transmitted between the surface of the heater 30 and the contact surface of the cigarette 7, so that the residue attached to the surface of the heater 30 is separated from the surface of the heater 30.

After the heater 30 is first separated from the cigarette 7 through the above-described operation, the user may grab the cigarette 7 to separate the cigarette 7 from the aerosol generating apparatus to easily discharge the residual attached to the cigarette 7 together with the cigarette 7 to the outside of the aerosol generating apparatus.

If there is no elastic support part 40 for supporting the container 20, the user pressing the cigarette 7 cannot know whether the cigarette 7 is completely inserted into the container 20, so that the user may continuously press the cigarette 7 with excessive force.

However, in the aerosol generating apparatus according to the above-described embodiment, while the user presses the cigarette 7, the resistance force by the elastic support part 40 is applied to the container 20 moving along the case 10. Therefore, the user may know that the cigarette 7 is completely inserted into the container 20 by feeling the resistance force transmitted from the container 20 and the cigarette 7.

When the user who has pressed the container 20 and the cigarette 7 releases the cigarette 7, the cigarette 7 and the container 20 move linearly toward the upper side, and the cigarette 7 and the container 20 move to the initial position as illustrated in FIGS. 2 and 3. In the state illustrated in FIGS. 2 and 3, the heater 30 heats the cigarette 7 to generate the cigarette smoke.

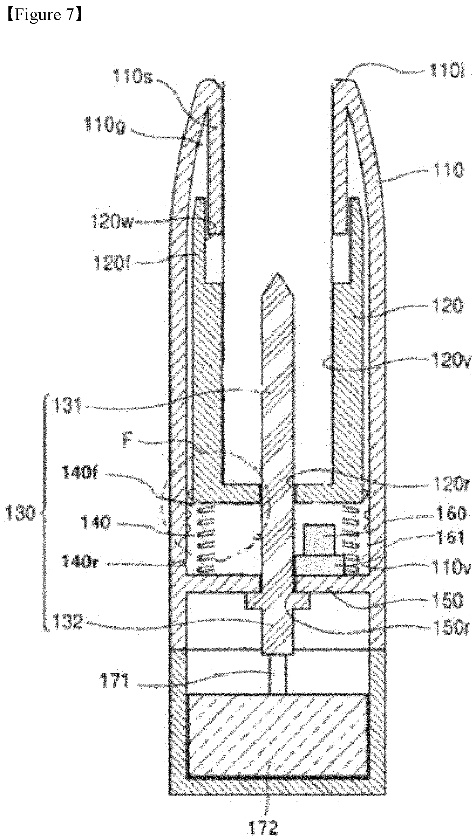

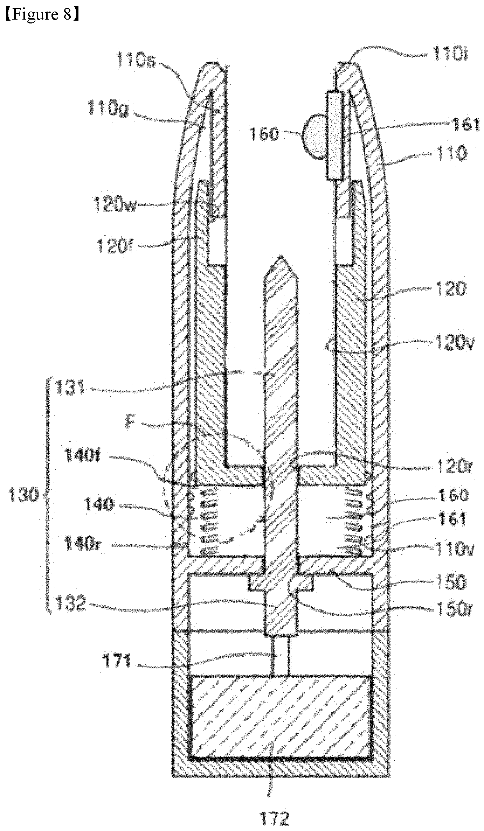

FIGS. 7 and 8 are cross-sectional views of an aerosol generating apparatus according to another embodiment.

An aerosol generating apparatus according to the embodiment illustrated in FIGS. 7 to 8 includes a case 110, a container 120 installed inside the case 110 so as to be movable in a longitudinal direction of the case 110 and including a receiving space 120v receiving a cigarette 7, a heater 130 disposed inside the case 110 so that a front end 131 is inserted into the receiving space 120v of the container 120 and heating the cigarette 7, and an elastic support part 140 supporting elastically the container 120 to the case 110.

The case 110 forms an appearance of the aerosol generating apparatus and performs a function of receiving and protecting various components in an inner space 110v formed therein.

The case 110 has a hollow cylindrical shape with an empty inside, and has an opening 110i into which the cigarette 7 may be inserted by opening to the outside at the front end. The case 110 may be made of a plastic material that does not transfer electricity and heat or a metallic material coated with a plastic material on the surface.

The container 120 is installed inside the case 110 so as to be movable in the longitudinal direction of the case 110. The cigarette 7 may be formed in a cylindrical shape, and the container 120 and the case 110 also extend in the longitudinal direction of the cigarette 7 in response to the shape of the cigarette 7.

The container 120 is formed in a hollow cylindrical shape, and includes an opening opened in a front end so that the cigarette 7 is inserted, a through hole 120r through which the front end 131 of the heater 130 passes at a rear end, and a receiving space 120v receiving the cigarette 7 therein.

The container 120 receives and supports the cigarette 7 while received inside the case 110 and performs a function of moving in the longitudinal direction of the case 110 together with the cigarette 7. Accordingly, the container 120 is not separated from the outside of the case 110.

The heater 130 that performs a function of heating the cigarette 7 is disposed inside the case 110. The front end 131 of the heater 130 is inserted into the inside of the container 120 through the through hole 120r of the container 120, and when the cigarette 7 is received in the container 120, the front end 131 of the heater 130 is inserted into the rear end of the cigarette 7.

A rear end 132 of the heater 130 is electrically connected to an electricity supply device 72 disposed at the rear side of the case 110 through an electric wiring 171. When the electricity of the electricity supply device 172 is supplied to the heater 130 while the cigarette 7 is inserted into the front end 131 of the heater 130, the heater 130 is heated to heat the cigarette 7.

The case 110 includes a fixing part 150 that is coupled to the rear end 132 of the heater 130 to fix the position of the heater 130 to the case 110. The fixing part 150 includes a space 150v having a hollow cylindrical shape with an opened upper end and receiving the container 120 therein. The fixing part 150 includes a hole 150r to which the front end 131 of the heater 130 is inserted at the rear end.

The front end 131 of the heater 130 passes through the hole 150r of the fixing part 150. The heater 130 includes a flange protruding from the outer surface. The position of the heater 130 may be fixed to the case 110 by fixing the flange to the fixing part 150.

The elastic support part 140 is disposed between the fixing part 150 and the container 120 to perform a function of elastically supporting the container 120 with respect to the case 110. In the illustrated embodiment, the elastic support part 140 has been implemented by a compression coil spring having a cylindrical cross-sectional shape, but the embodiment is not limited to the example of the elastic support part 140. For example, the elastic support part 140 may be implemented by a compression cylinder using liquid or gas, rubber, or the like.

The container 120 includes an extension part 120f in which an inner diameter of one end is extended outward. The inner diameter of the extension part 120f is formed larger than the outer diameter of the cigarette 7. The case 110 includes an insertion part 110s which is inserted between an inner wall surface 120w of the extension part 120f of the container 120 and the outer circumferential surface of the cigarette 7 to extend linearly in a moving direction of the container 120. That is, while the extension part 120f of the container 120 is inserted into a guide space 110g formed between the extension part 120f of the case 110 and the inner wall surface 120w of the case 110, the container 120 may move linearly in a longitudinal direction of the case 110.

One end 140f of the elastic support part 140 is supported by a lower surface of the container 120 and the other end 140r is supported by the fixing part 150.

The switch 160 includes a sensor 161 that receives an operation signal of the switch to apply electricity to the heater 130. The sensor 161 may be a micro controller unit (MCU), but is not limited thereto.

The switch 160 is disposed between the fixing part 150 and the elastic support part 140 as illustrated in FIG. 7, or may be disposed between the insertion part 110s and the outer circumferential surface of the cigarette 7 as illustrated in FIG. 8.

The operation state of pressing the cigarette to insert or remove the cigarette in the aerosol generating apparatus of another embodiment is the same as that of the one embodiment described above. At this time, the operation state in which the switch 160 detects whether the cigarette is inserted or not and the state in which the sensor 161 receives the operation signal of the switch 160 to apply the electricity to the heater and then stops the heating or the application of the electricity to stop the heating are the same as those of the one embodiment described above.

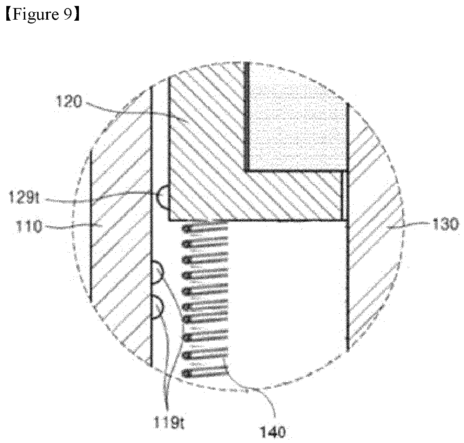

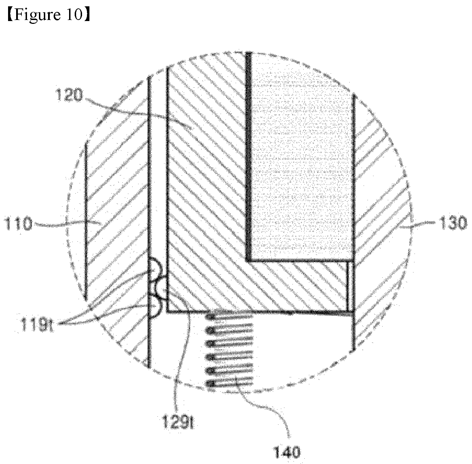

FIG. 9 is a cross-sectional view illustrating an operating state of the aerosol generating apparatus according to the embodiment illustrated in FIGS. 7 and 8. FIGS. 9 and 10 enlarge and illustrate a portion represented by F of FIGS. 7 and 8.

The aerosol generating apparatus has a stopper disposed between the container 120 and the case 110 to impart the resistance force against the movement of the container 120. The stopper has a moving protrusion 129t protruding from an outer surface of the container 120 and a fixing protrusion 119t protruding from an inner surface of the case 110.

When the user presses the container 120 downward, as illustrated in FIG. 6, the moving protrusion 129t of the container 120 moves downward together with the container 120 to approach the fixing protrusion 119t.

When the user continuously presses the container 120, as illustrated in FIG. 7, the moving protrusion 129t of the container 120 is inserted between the fixing protrusion 119t of the case 110, so that the movement of the container 120 is limited by a coupling action of the moving protrusion 129t and the fixing protrusion 119t.

When a force of fixing the moving protrusion 129t by two fixing protrusions 119t is set to be larger than the pressing force of the elastic support part 140 by largely forming the sizes of the moving protrusion 129t and the fixing protrusion 119t, the stopper may perform a function of maintaining the container 120 in the pressing position illustrated in FIG. 10. In this case, in order to move the container 120 upward from the pressing position illustrated in FIG. 10, the user needs to release the coupling of the moving protrusion 129t and the fixing protrusion 119t by applying a force to the container 120. When the moving protrusion 129t is removed from the fixing protrusion 119t, the container 120 moves upward by a restoring force of the elastic support part 140.

When a force of fixing the moving protrusion 129t by two fixing protrusions 119t is set to be smaller than the pressing force of the elastic support part 140 by largely forming the sizes of the moving protrusion 129t and the fixing protrusion 119t, the stopper may not perform the function of maintaining the container 120 in the pressing position illustrated in FIG. 10, and performs only a function of imparting the resistance force to the movement of the container 120 which has moved downward. That is, while the container 120 reaches the pressing position illustrated in FIG. 10 and then the moving protrusion 129t is inserted between the two fixing protrusions 119t, the resistance is transmitted to a hand of the user who has pressed the container 120, so that the user may determine that the container 120 is sufficiently pressed.

In the above-described embodiment, the two fixing protrusions 119t have been illustrated, but the embodiment is not limited by the number, the shape, or the size of the fixing protrusion 119t. For example, only one fixing protrusion 119t is installed, but the moving protrusion 129t and the fixing protrusion 119t may perform a function of limiting the linear movement of the container 120 so that the container 120 cannot move further over the pressing position by making the sizes of the moving protrusion 129t and the fixing protrusion 119t large sufficiently so that the moving protrusion 129t cannot pass through the fixing protrusion 119t.

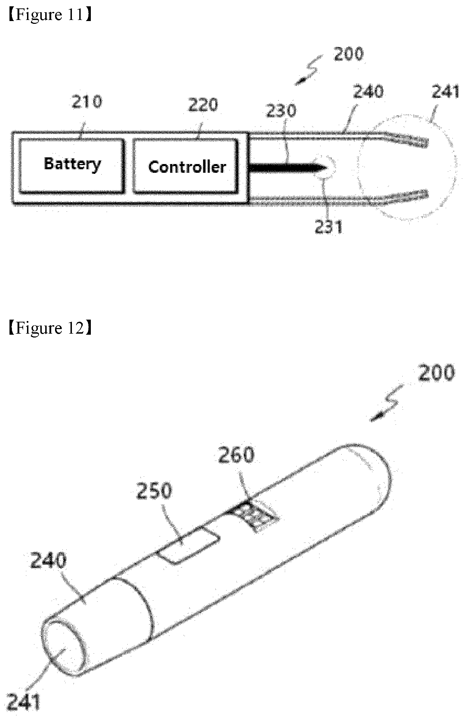

Embodiments illustrated in FIGS. 11 to 13 below illustrate a modified aerosol generating apparatus that may be applied to the aerosol generating apparatus according to the embodiments illustrated in FIGS. 1 to 10 described above.

FIG. 11 is a configuration diagram illustrating an example of an aerosol generating apparatus.

Referring to FIG. 11, an aerosol generating apparatus 1 (hereinafter, referred to as a 'holder') includes a battery 210, a controller 220, and a heater 230. In addition, the holder 1 includes an inner space formed by a case 240. A cigarette may be inserted into the inner space of the holder 1.

Only components associated with the embodiment are illustrated in the holder 1 illustrated in FIG. 11. Accordingly, it may be understood to those skilled in the art associated with the embodiment that other universal components other than the components illustrated in FIG. 11 may be further included in the holder 1.

When the cigarette is inserted into the holder 1, the holder 1 heats the heater 230. The temperature of an aerosol generating material in the cigarette rises by the heated heater 230, and as a result, an aerosol is generated. The generated aerosol is transmitted to the user through a filter of the cigarette. However, even when the cigarette is not inserted into the holder 1, the holder 1 may heat the heater 230.

The case 240 may be separated from the holder 1. For example, when the user rotates the case 240 in a clockwise or counterclockwise direction, the case 240 may be separated from the holder 1.

In addition, the diameter of a hole formed by an end 241 of the case 240 may be made smaller than the diameter of a space formed by the case 240 and the heater 230, and in this case, the hole may serve as a guide of the cigarette to be inserted to the holder 1.

The battery 210 supplies power used for operating the holder 1. For example, the battery 210 may supply power so that the heater 230 may be heated, and may supply power required for operating the controller 220. In addition, the battery 210 may supply power required for operating a display, a sensor, a motor, and the like installed in the holder 1.

The battery 210 may be a lithium iron phosphate (LiFePO$_4$) battery, but is not limited to the above-described example. For example, the battery 210 may correspond to a lithium cobalt oxide (LiCoO$_2$) battery, a lithium titanate battery, or the like.

Further, the battery 210 may have a cylindrical shape having a diameter of 10 mm and a length of 37 mm, but is not limited thereto. The capacity of the battery 210 may be 120 mAh or more, and may be a rechargeable battery or a disposable battery. For example, when the battery 210 is rechargeable, a charging rate (C-rate) of the battery 210 may be 10 C, and a discharging rate (C-rate) of the battery 110 may be 16 C to 20 C, but is not limited thereto. In addition, for stable use, the battery 210 may be manufactured so that 80% or more of the total capacity may be secured even when charging/discharging proceeds 8000 times.

Here, whether the battery 210 is fully charged or discharged may be determined by the level of power stored in the battery 210 based on the total capacity of the battery 210. For example, when the power stored in the battery 210 is 95% or more of the total capacity, it may be determined that the battery 210 is fully charged. In addition, when the power stored in the battery 210 is 10% or less of the total capacity, it may be determined that the battery 210 is fully discharged. However, a standard for determining whether the battery 210 is fully charged and discharged is not limited to the above-described example.

The heater 230 is heated by the power supplied from the battery 210. When the cigarette is inserted into the holder 1, the heater 230 is positioned inside the cigarette. Accordingly, the heated heater 230 may raise the temperature of the aerosol generating material in the cigarette.

The heater 230 may have a shape in which a cylinder and a cone are combined. For example, the heater 230 has a cylindrical shape having a diameter of about 2 mm and a length of about 23 mm, and the end 231 of the heater 230 may be finished at an acute angle, but is not limited thereto. In other words, the heater 230 may correspond to any form which may be inserted into the cigarette without limitation. In addition, only a portion of the heater 230 may also be heated. For example, if the length of the heater 230 is 23 mm, only 12 mm from the end 231 of the heater 230 is heated, and the remaining portion of the heater 230 may not be heated.

The heater 230 may be an electric resistive heater. For example, the heater 230 may include an electrically conductive track, and as the current flows through the electrically conductive track, the heater 230 may be heated.

For stable use, the heater 230 may be supplied with power according to the specifications of 3.2 V, 2.4 A, and 8 W, but is not limited thereto. For example, when the power is supplied to the heater 230, the surface temperature of the heater 230 may rise to 400°C or higher.

The surface temperature of the heater 230 may rise to about 350°C before 15 seconds are exceeded from when the power starts to be supplied to the heater 230.

A separate temperature sensor may be provided in the holder 1. Alternatively, the holder 1 is not provided with a temperature sensor, and the heater 230 may also serve as a temperature sensor. For example, the heater 230 may further include a second electrically conductive track for sensing the temperature in addition to the first electrically conductive track for generating the heat.

For example, when a voltage applied to the second electrically conductive track and a current flowing through the second electrically conductive track are measured, a resistance R may be determined. At this time, a temperature T of the second electrically conductive track may be determined by Equation 1 below.

$$[\text{Equation 1}]$$
$$R = R_0\{1 + \alpha(T - T_0)\}$$

In Equation 1, R means a current resistance value of the second electrically conductive track, $R_0$ means a resistance value at a temperature $T_0$ (e.g. 0°C), and $\alpha$ means a resistance temperature coefficient of the second electrically conductive track. A conductive material (e.g. metal) has an intrinsic resistance temperature coefficient, and $\alpha$ may be predetermined depending on a conductive material constituting the second electrically conductive track. Accordingly, when the resistance R of the second electrically conductive track is determined, the temperature T of the second electrically conductive track may be calculated by Equation 1 above.

The heater 230 may be constituted by at least one electrically conductive track (the first electrically conductive track and the second electrically conductive track). For example, the heater 230 may be constituted by two first electrically conductive tracks and one or two second electrically conductive tracks, but is not limited thereto.

The electrically conductive track includes an electrically resistive material. As an example, the electrically conductive track may be made of a metallic material. As another example, the electrically conductive track may be made of an electrically conductive ceramic material, carbon, a metal alloy, or a composite material of a ceramic material and metal.

Further, the holder 1 may include both an electrically conductive track that serves as a temperature sensor and a temperature sensor.

The controller 220 generally controls the operation of the holder 1. Specifically, the controller 220 controls the operations of other components included in the holder 1 as well as the battery 210 and the heater 230. In addition, the controller 220 may determine whether the holder 1 is operable by checking a state of each of the components of the holder 1.

The controller 220 includes at least one processor. The processor may be implemented by an array of multiple logic gates, or may also be implemented in a combination of a general-purpose microprocessor and a memory in which programs that may be executed in the microprocessor are stored. In addition, it may be understood to those skilled in the art to which the embodiment pertains that the processor may be implemented with other types of hardware.

For example, the controller 220 may control the operation of the heater 230. The controller 220 may control an amount of power to be supplied to the heater 230 and a time at which the power is to be supplied so that the heater 230 may be heated to a predetermined temperature or maintained at an appropriate temperature.

In addition, the controller 220 may check the state of the battery 210 (e.g. the remaining amount of the battery 210, etc.), and generate an alarm signal if necessary.

In addition, the controller 220 may check the presence or absence of the puff of the user and the strength of the puff, and count the number of puffs. In addition, the controller 220 may continuously check the time that the holder 1 is operating. In addition, the controller 220 checks whether the cradle 2 to be described below is coupled with the holder 1, and may control the operation of the holder 1 according to the coupling or separation of the cradle 2 and the holder 1.

Meanwhile, the holder 1 may further include general-purpose components in addition to the battery 210, the controller 220, and the heater 230.

For example, the holder 1 may include a display capable of outputting visual information or a motor for outputting tactile information. As an example, when the display is included in the holder 1, the controller 220 may deliver, to the user through the display, information about the state of the holder 1 (e.g. whether the holder is usable, etc.), information about the heater 230 (e.g. start of preheating, progression of preheating, completion of preheating, etc.), information about the battery 210 (e.g. a remaining capacity of the battery 210, availability, etc.), information about reset of the holder 1 (e.g. reset timing, reset progression, reset completion, etc.), information about cleaning of the holder 1 (e.g. cleaning timing, need of cleaning, progression of cleaning, completion of cleaning, etc.), information about charging of the holder 1 (e.g. need of charging, progression of charging, completion of charging, etc.), information about the puff (e.g. the number of puffs, notice of puff termination, etc.), or information about safety (e.g. usage time elapsed, etc.). As another example, when the holder 1 includes the motor, the controller 220 may transmit the above-described information to the user by generating a vibration signal using the motor.

In addition, the holder 1 may include at least one input device (e.g., a button) that allows the user to control the function of the holder 1 and/or a terminal coupled with a cradle 2. For example, the user may perform various functions using the input device of the holder 1. The user may perform a desired function of multiple functions of the holder 1 by adjusting the number of times of pressing the input device (e.g. once, twice, etc.) or the time of pressing the input device (e.g. 0.1 sec, 0.2 sec, etc.). As the user operates the input device, the holder 1 may perform a function of preheating the heater 230, a function of adjusting the temperature of the heater 230, a function of cleaning a space into which the cigarette is inserted, a function of checking whether the holder 1 is in an operable state, a function of displaying the remaining amount (available power) of the battery 210, a reset function of the holder 1, and the like. However, the function of the holder 1 is not limited to the examples described above.

Further, the holder 1 may include a puff sensor or a temperature sensor. For example, the puff sensor may be implemented by a general pressure sensor. In addition, the holder 1 may be manufactured in a structure that allows external air to flow in/out even when the cigarette is inserted.

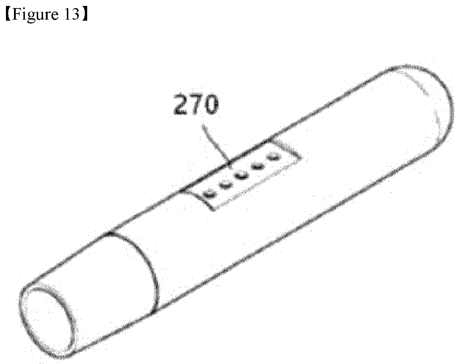

FIGS. 12 and 13 are diagrams illustrating an example of the holder in various aspects.

FIG. 12 is a diagram illustrating an example of the holder 1 viewed from a first direction. As illustrated in FIG. 12, the holder 1 may be formed in a cylindrical shape, but is not limited thereto. The case 240 of the holder 1 may be separated by an operation of the user, and the cigarette may be inserted into the end 241 of the case 240. In addition, the holder 1 may include a button 250 by which a user may control the holder 1 and a display 260 on which an image is output.

FIG. 13 is a diagram illustrating an example of the holder 1 viewed from a second direction. The holder 1 may include a terminal 270 coupled with the cradle 2. Since the terminal 270 of the holder 1 is coupled with the terminal of the cradle 2, the battery 210 of the holder 1 may be charged by the power supplied by the battery 310 of the cradle 2. Further, through the terminal 270 and the terminal, the holder 1 may be operated by the power supplied by the battery 310 of the cradle 2, and communication between the holder 1 and the cradle 2 (signal transmission and reception) is enabled. For example, the terminal 270 may be constituted by four micro pins, but is not limited thereto.

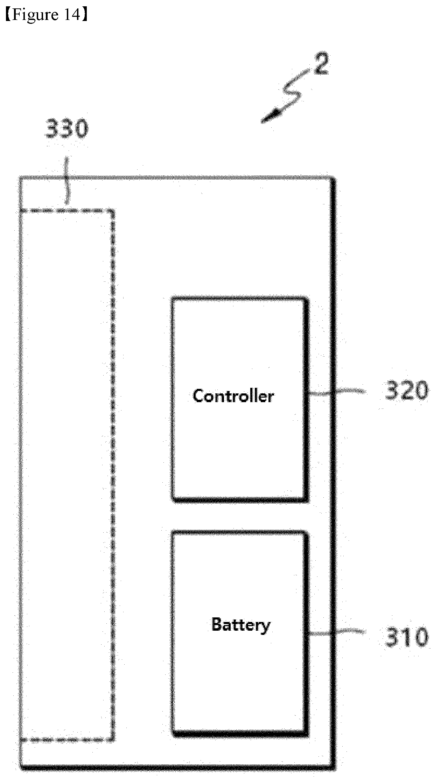

FIG. 14 is a configuration diagram illustrating an example of the cradle.

Referring to FIG. 14, the cradle 2 includes a battery 310 and a controller 320. In addition, the cradle 2 includes an inner space 330 into which the holder 1 may be inserted. For example, the inner space 330 may be formed on one side of the cradle 2. Therefore, even if the cradle 2 does not include a separate lid, the holder 1 may be inserted and fixed into the cradle 2.

Only components associated with the embodiment are illustrated in the cradle 2 illustrated in FIG. 14. Accordingly, it may be understood to those skilled in the art associated with the embodiment that other universal components other than the components illustrated in FIG. 14 may be further included in the cradle 2.

The battery 310 supplies power used for operating the cradle 2. In addition, the battery 310 may supply power for charging the battery 210 of the holder 1. For example, when the holder 1 is inserted into the cradle 2 so that the terminal 270 of the holder 1 and the terminal of the cradle 2 are coupled to each other, the battery 310 of the cradle 2 may supply power to the battery 210 of the holder 1.

In addition, when the holder 1 and the cradle 2 are coupled to each other, the battery 310 may supply power used for operating the holder 1. For example, when the terminal 270 of the holder 1 and the terminal of the cradle 2 are coupled with each other, regardless of whether the battery 210 of the holder 1 is discharged, the holder 1 may operate using the power supplied by the battery 310 of the cradle 2.

An example of the type of the battery 310 may be the same as the example of the battery 210 described above with reference to FIG. 11. The capacity of the battery 310 may be larger than the capacity of the battery 210, for example, the capacity of the battery 310 may be 3000 mAh or more, but the capacity of the battery 310 is not limited to the above-described example.

The controller 320 generally controls the operation of the cradle 2. The controller 320 may control operations of all components of the cradle 2. In addition, the controller 320 determines whether the cradle 2 is coupled with the holder 1, and may control the operation of the cradle 2 according to the coupling or separation of the cradle 2 and the holder 1.

For example, when the holder 1 and the cradle 2 are coupled with each other, the controller 320 may charge the battery 210 or heat the heater 230 by supplying the power of the battery 310 to the holder 1. Therefore, even when the remaining amount of the battery 210 is small, the user may continuously smoke by coupling the holder 1 and the cradle 2.

The controller 220 includes at least one processor. The processor may be implemented by an array of multiple logic gates, or may also be implemented in a combination of a general-purpose microprocessor and a memory in which programs that may be executed in the microprocessor are stored. In addition, it can be understood to those skilled in the art to which the embodiment pertains that the processor may be implemented by other types of hardware.

Meanwhile, the cradle 2 may further include general-purpose components in addition to the battery 310 and the controller 320. For example, the cradle 2 may include a display capable of outputting visual information. For example, when the cradle 2 includes the display, the controller 320 may generate a signal to be displayed on the display to deliver to the user information about the battery 320 (e.g. a remaining capacity of the battery 320, availability, etc.), information about reset of the cradle 2 (e.g. reset timing, reset progression, reset completion, etc.), information about cleaning of the holder 1 (e.g. cleaning timing, need of cleaning, cleaning progression, cleaning completion, etc.), and information about charging of the cradle 2 (e.g. need of charging, charging progression, charging completion, etc.).

In addition, the cradle 2 may also include at least one input device (e.g. a button) which allows the user to control the function of the cradle 2, a terminal coupled with the holder 1, and/or an interface (e.g. a USB port, etc.) for charging the battery 310.

For example, the user may perform various functions using the input device of the cradle 2. The user may execute a desired function of a plurality of functions of the cradle 2 by adjusting the number of times of pressing the input device or the time of pressing the input device. As the user operates the input device, the cradle 2 may perform a function of preheating the heater 230 of the holder 1, a function of adjusting the temperature of the heater 230 of the holder 1, a function of cleaning a space in the holder 1 into which the cigarette is inserted, a function of checking whether the cradle 2 is in an operable state, a function of displaying the remaining amount (available power) of the battery 310 of the cradle 2, a reset function of the cradle 2, and the like. However, the functions of the cradle 2 are not limited to the examples described above.

[Description of Symbols]

7: Cigarette
10g, 110g: Guide space
10i, 110i: Opening
10s, 110s: Insertion part
10v, 110v: Inner space
10, 110, 240: Case
19: Base
20w, 120w: Inner wall surface
20v, 120v: Receiving space
20, 120: Container
20r, 120r: Through hole
20f, 120f: Extension part
29: Support projection
30, 130, 230: Heater
30p: Flange
31, 131: Front end
32, 132: Rear end
40r, 140r: The other end
40f, 140f: One end
40, 140: Elastic support part
50, 150: Fixing part
50r, 150r: Hole
50v, 150v: Space
60, 160: Switch
61, 161: Sensor
71: Electric wiring
72: Electricity supply device
119t: Fixing protrusion
129t: Moving protrusion
210: Battery of holder
220: Controller of holder
231: End of heater
241: End of case

250: Button
260: Display
270: Terminal
310: Battery of cradle
320: Controller of cradle
330: Inner space of cradle

**Claims**

1. An aerosol generating apparatus comprising:

   a case (10, 110, 240);
   a container (20, 120) which is installed inside the case (10, 110, 240) to move in a longitudinal direction of the case (10, 110, 240) and includes a receiving space (20v, 120v) for receiving a cigarette (7);
   a heater (30, 130, 230) which is disposed inside the case (10, 110, 240) so that a front end (31, 131) is inserted into the receiving space (20v, 120v) of the container (20, 120) and heats the cigarette (7) when electricity is applied; and
   an elastic support part (40, 140) which elastically supports the container (20, 120) with respect to the case (10, 110, 240);

   **characterized in that**:

   the aerosol generating apparatus further comprises: a switch (60, 160) which detects whether the cigarette (7) is inserted or not to apply electricity to the heater (30, 130, 230); and
   wherein the container (20, 120) further comprises an extension part (20f, 120f) in which an inner diameter of one end extends outward, and the case (10, 110, 240) further comprises an insertion part (10s, 110s) which is inserted between an inner wall surface of the extension part (20f, 120f) of the container (20, 120) and an outer circumferential surface of the cigarette (7) to extend linearly in a movement direction of the container (20, 120).

2. The aerosol generating apparatus of claim 1, wherein the switch (60, 160) comprises a sensor (61, 161) which receives an operation signal of the switch (60, 160) to apply the electricity to the heater (30, 130, 230).

3. The aerosol generating apparatus of claim 2, wherein the switch (60, 160) is in an on state when the cigarette (7) is inserted and the sensor (61, 161) receives an operation signal in the on state of the switch (60, 160) to apply the electricity to the heater (30, 130, 230) and heats the heater (30, 130, 230), and
   The switch (60, 160) is in an off state when the cigarette (7) is removed and the sensor (61, 161) receives an operation signal in the off state of the switch (60, 160) to stop the application of electricity to the heater (30, 130, 230) and terminate the heating.

4. The aerosol generating apparatus of claim 3, wherein the switch (60, 160) is installed inside the case (10, 110, 240) which guides the insertion of the cigarette (7) when the cigarette (7) is inserted, an inner wall surface (20w, 120w) of the container (20, 120), or at a position which is pressed by a bottom surface of the container (20, 120) at the time of completing the movement of the container (20, 120).

5. The aerosol generating apparatus of claim 1, further comprising: a fixing part (50, 150) which is coupled to a rear end (32, 132) of the heater (30, 130, 230) and fixes the position of the heater (30, 130, 230) to the case (10, 110, 240), wherein the elastic support part (40, 140) is disposed between the fixing part (50, 150) and the container (20, 120).

6. The aerosol generating apparatus of claim 5, wherein the container (20, 120) further comprises a support projection (29) formed on a surface facing the fixing part (50, 150) at the outside of the extension part (20f, 120f) and the elastic support part (40, 140) is disposed between the support projection (29) and the fixing part (50, 150).

7. The aerosol generating apparatus of claim 5, wherein the switch (60, 160) is installed between the fixing part (50, 150) and the container (20, 120).

8. The aerosol generating apparatus of claim 5, wherein the switch (60, 160) is disposed in the insertion part (10s, 110s) to face the outer circumferential surface of the cigarette (7).

**Patentansprüche**

1. Aerosolerzeugungsvorrichtung, die Folgendes umfasst:

   ein Gehäuse (10, 110, 240);
   einen Behälter (20, 120), der im Gehäuse (10, 110, 240) installiert ist, der sich in einer Längsrichtung des Gehäuses (10, 110, 240) bewegt und einen Aufnahmeraum (20v, 120v) zum Aufnehmen einer Zigarette (7) umfasst;
   eine Heizvorrichtung (30, 130, 230), die so im Gehäuse (10, 110, 240) angeordnet ist, dass ein vorderes Ende (31, 131) in den Aufnahmeraum (20v, 120v) des Behälters (20, 120) eingesetzt ist und die Zigarette (7) erhitzt, wenn Elektrizität zugeführt wird; und
   ein elastisches Tragteil (40, 140), das den Behälter (20, 120) in Bezug auf das Gehäuse (10, 110, 240) elastisch trägt;
**dadurch gekennzeichnet, dass**:

die Aerosolerzeugungsvorrichtung ferner Folgendes umfasst: einen Schalter (60, 160), der detektiert, ob die Zigarette (7) eingesetzt ist, um der Heizvorrichtung (30, 130, 230) Elektrizität zuzuführen; und wobei der Behälter (20, 120) ferner ein Verlängerungsteil (20f, 120f) umfasst, bei dem sich ein Innendurchmesser eines Endes nach außen erweitert, und wobei das Gehäuse (10, 110, 240) ferner ein Einsetzteil (10s, 110s) umfasst, das zwischen einer Innenwandfläche des Verlängerungsteils (20f, 120f) des Behälters (20, 120) und einer Außenumfangsfläche der Zigarette (7) eingesetzt ist, so dass es sich in einer Bewegungsrichtung des Behälters (20, 120) geradlinig erstreckt.

2. Aerosolerzeugungsvorrichtung nach Anspruch 1, wobei der Schalter (60, 160) einen Sensor (61, 161) umfasst, der ein Betriebssignal des Schalters (60, 160) erhält, um der Heizvorrichtung (30, 130, 230) Elektrizität zuzuführen.

3. Aerosolerzeugungsvorrichtung nach Anspruch 2, wobei der Schalter (60, 160) in einem Ein-Zustand ist, wenn die Zigarette (7) eingesetzt ist und der Sensor (61, 161) ein Betriebssignal im Ein-Zustand des Schalters (60, 160) erhält, um der Heizvorrichtung (30, 130, 230) Elektrizität zuzuführen und die Heizvorrichtung (30, 130, 230) zu heizen, und der Schalter (60, 160) in einem Aus-Zustand ist, wenn die Zigarette (7) entfernt worden ist und der Sensor (61, 161) ein Betriebssignal im Aus-Zustand des Schalters (60, 160) erhält, um das Zuführen von Elektrizität zur Heizvorrichtung (30, 130, 230) zu stoppen und das Heizen zu beenden.

4. Aerosolerzeugungsvorrichtung nach Anspruch 3, wobei der Schalter (60, 160) im Gehäuse (10, 110, 240), das das Einsetzen der Zigarette (7) führt, wenn die Zigarette (7) eingesetzt wird, an einer Innenwandfläche (20w, 120w) des Behälters (20, 120) oder an einer Position, die durch eine Bodenfläche des Behälters (20, 120) zu dem Zeitpunkt des Beendens der Bewegung des Behälters (20, 120) gepresst wird, installiert ist.

5. Aerosolerzeugungsvorrichtung nach Anspruch 1, die ferner Folgendes umfasst: ein Fixierteil (50, 150), das mit einem hinteren Ende (32, 132) der Heizvorrichtung (30, 130, 230) gekoppelt ist und die Position der Heizvorrichtung (30, 130, 230) am Gehäuse (10, 110, 240) fixiert, wobei das elastische Tragteil (40, 140) zwischen dem Fixierteil (50, 150) und dem Behälter (20, 120) angeordnet ist.

6. Aerosolerzeugungsvorrichtung nach Anspruch 5, wobei der Behälter (20, 120) ferner einen Tragvorsprung (29) umfasst, der an einer Oberfläche ausgebildet ist, die an der Außenseite des Verlängerungsteils (20f, 120f) zum Fixierteil (50, 150) zeigt, und wobei das elastische Tragteil (40, 140) zwischen dem Tragvorsprung (29) und dem Fixierteil (50, 150) angeordnet ist.

7. Aerosolerzeugungsvorrichtung nach Anspruch 5, wobei der Schalter (60, 160) zwischen dem Fixierteil (50, 150) und dem Behälter (20, 120) installiert ist.

8. Aerosolerzeugungsvorrichtung nach Anspruch 5, wobei der Schalter (60, 160) im Einsetzteil (10s, 110s) so angeordnet ist, dass er zur Außenumfangsfläche der Zigarette (7) zeigt.

**Revendications**

1. Appareil de production d'aérosol comportant :

un étui (10, 110, 240) ;
un contenant (20, 120) qui est installé à l'intérieur de l'étui (10, 110, 240) de manière à se déplacer dans une direction longitudinale de l'étui (10, 110, 240) et inclut un espace de réception (20v, 120v) destiné à recevoir une cigarette (7) ;
un élément chauffant (30, 130, 230) qui est disposé à l'intérieur de l'étui (10, 110, 240) de sorte qu'une extrémité avant (31, 131) est insérée dans l'espace de réception (20v, 120v) du contenant (20, 120) et chauffe la cigarette (7) lorsque de l'électricité est appliquée ; et
une partie de support élastique (40, 140) qui supporte élastiquement le contenant (20, 120) par rapport à l'étui (10, 110, 240) ;
**caractérisé en ce que** :

l'appareil de production d'aérosol comporte en outre : un commutateur (60, 160) qui détecte si la cigarette (7) est insérée ou non afin d'appliquer de l'électricité à l'élément chauffant (30, 130, 230) ; et
dans lequel le contenant (20, 120) comporte en outre une partie d'extension (20f, 120f) dans laquelle un diamètre intérieur d'une extrémité s'étend vers l'extérieur, et l'étui (10, 110, 240) comporte en outre une partie d'insertion (10s, 110s) qui est insérée entre une surface de paroi intérieure de la partie d'extension (20f, 120f) du contenant (20, 120) et une surface circonférentielle extérieure de la cigarette (7) pour s'étendre linéairement dans une direction de déplacement du contenant (20, 120).

2. Appareil de production d'aérosol selon la revendication 1, dans lequel le commutateur (60, 160) comporte un capteur (61, 161) qui reçoit un signal de fonctionnement du commutateur (60, 160) afin d'appliquer l'électricité à l'élément chauffant (30, 130, 230).

3. Appareil de production d'aérosol selon la revendication 2, dans lequel le commutateur (60, 160) est dans un état passant lorsque la cigarette (7) est insérée et le capteur (61, 161) reçoit un signal de fonctionnement dans l'état passant du commutateur (60, 160) afin d'appliquer l'électricité à l'élément chauffant (30, 130, 230) et chauffe l'élément chauffant (30, 130, 230), et
le commutateur (60, 160) est dans un état bloqué lorsque la cigarette (7) est retirée et le capteur (61, 161) reçoit un signal de fonctionnement dans l'état bloqué du commutateur (60, 160) afin d'arrêter l'application d'électricité à l'élément chauffant (30, 130, 230) et de mettre fin au chauffage.

4. Appareil de production d'aérosol selon la revendication 3, dans lequel le commutateur (60, 160) est installé à l'intérieur de l'étui (10, 110, 240) qui guide l'insertion de la cigarette (7) lorsque la cigarette (7) est insérée, une surface de paroi intérieure (20w, 120w) du contenant (20, 120), ou à une position qui est pressée par une surface de fond du contenant (20, 120) au moment de la fin du mouvement du contenant (20, 120).

5. Appareil de production d'aérosol selon la revendication 1, comportant en outre : une partie de fixation (50, 150) qui est couplée à une extrémité arrière (32, 132) de l'élément chauffant (30, 130, 230) et fixe la position de l'élément chauffant (30, 130, 230) à l'étui (10, 110, 240), dans lequel la partie de support élastique (40, 140) est disposée entre la partie de fixation (50, 150) et le contenant (20, 120).

6. Appareil de production d'aérosol selon la revendication 5, dans lequel le contenant (20, 120) comporte en outre une saillie de support (29) formée sur une surface dirigée vers la partie de fixation (50, 150) sur l'extérieur de la partie d'extension (20f, 120f) et la partie de support élastique (40, 140) est disposée entre la saillie de support (29) et la partie de fixation (50, 150).

7. Appareil de production d'aérosol selon la revendication 5, dans lequel le commutateur (60, 160) est installé entre la partie de fixation (50, 150) et le contenant (20, 120).

8. Appareil de production d'aérosol selon la revendication 5, dans lequel le commutateur (60, 160) est disposé dans la partie d'insertion (10s, 110s) pour faire face à la surface circonférentielle extérieure de la cigarette (7).

【Figure 1】

【Figure 2】

【Figure 3】

【Figure 4】

【Figure 5】

【Figure 6】

【Figure 7】

【Figure 8】

【Figure 9】

【Figure 10】

【Figure 11】

【Figure 12】

【Figure 13】

【Figure 14】

**EP 3 785 554 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101667124
- US 2017172215 A1

- WO 2016199066 A1